# EUROPEAN PATENT APPLICATION

(11) **EP 4 170 315 A1**
(43) Date of publication of application: **26.04.2023**
(21) Application number: 21306470.2
(22) Date of filing: 22.10.2021
(51) Int. Cl.: G01N 1/10, G01N 33/48

(54) **METHODS OF EVALUATING MICRO RNA**

(71) Applicant: Johnson & Johnson Consumer Inc., Skillman, NJ 08558 (US)
(72) Inventor: BRUN, Cécilia, 27100 Val de Reuil (FR); ROUX, Pierre-François, 27100 Val de Reuil (FR); MANGEZ, Claire, 27100 Val de Reuil (FR); ISON, Renny, 27100 Val de Reuil (FR); DONNELLY, Ryan F., Belfast, BT7 1NN (GB)
(74) Representative: Carpmaels & Ransford LLP

(57) **Abstract**

Provided are methods of quantifying miRNA in skin, the method comprising: applying a swellable microprotrusion array to a region of skin to absorb interstitial fluid; removing the microprotrusion array; recovering miRNA from the interstitial fluid absorbed into the microprotrusion array; and quantifying the miRNA. Also provided are methods of monitoring epigenetic changes in skin using a swellable microprotrusion array.

## Description

### FIELD

The present invention relates to methods of analyzing microRNA. More specifically, the present invention relates to methods using a swellable microprotrusion array for analyzing microRNA from skin samples.

### BACKGROUND

Epigenetics is a field which studies the heritable changes in gene expression which do not involve the alterations of the DNA sequence. This field is of great interest, as it may help to shed light on the effect of various environment and behavior factors on phenotype. One good biomarker for evaluating epigenetic effects is microRNA (miRNA). MiRNA are small non-coding RNA molecules having about 22 nucleotides, which play an important role in gene expression regulation.

Currently, the gold standard for *in vivo* epigenetic studies is the biopsy, whereby tissue is removed from a living body and then analyzed. However, there are considerable drawbacks to biopsies. Indeed, because biopsies are an invasive procedure, there are several limitations associated with the method. In particular, the invasive nature of the procedure limits studies on babies or on the face. Additionally, such invasive studies are usually much more expensive and longer to put in place as compared to noninvasive studies. Biopsies also need several processing steps to access epigenetic information. Some solutions have been proposed to detect biomarkers in a non-invasive manner, but there are no commercially available products that actually focus on miRNA. Collection devices are either complex or need probes. Additionally, any methods which could contemplate use with miRNAs are qualitative and not quantitative.

There is thus an ongoing need to identify new methods obtain quantitative data about epigenetic variation in the skin in a noninvasive way.

### SUMMARY OF THE INVENTION

One aspect of the invention pertains to a method of quantifying miRNA in skin, the method comprising:
applying a swellable microprotrusion array to a region of skin to absorb interstitial fluid; removing the microprotrusion array;
recovering miRNA from the interstitial fluid absorbed into the microprotrusion array; and
quantifying the miRNA.

In one or more embodiments, the microprotrusion array is applied to the skin for at least about 20 to about 180 minutes. In some embodiments, recovering the miRNA form the microprotrusion array comprises washing the microprotrusion array.

In one or more embodiments, the microprotrusion array is washed with a buffer selected from the group consisting of phosphate-buffered saline, distilled water, and an RNA stabilization reagent (such as the PAXgene^{®} Blood RNA Tube) to provide a washing liquid. In some embodiments, the method further comprises concentrating the washing liquid. In one or more embodiments, the microprotrusion array comprises a plurality of microprotrusions composed of a swellable polymer composition. In some embodiments, the swellable polymer composition is in its dry state hard and brittle sufficient to penetrate the stratum corneum of mammalian skin. In one or more embodiments, the method further comprises comparing the quantified miRNA to a reference. In some embodiments, quantifying the miRNA comprises next generation sequencing, transcriptome analysis, or quantitative polymerase chain reaction.

Another aspect of the invention pertains to method of monitoring epigenetic changes in skin as a result of exposure to a stimulus. In one or more embodiments, the method comprises
exposing a region of the skin of a subject to the stimulus;
applying a swellable microprotrusion array to at least a part of the region of skin to absorb interstitial fluid;
removing the microprotrusion array;
recovering miRNA from the microprotrusion array; and
analyzing the miRNA.

In some embodiments, the swellable microprotrusion array that is applied to at least a part of the region of skin to absorb interstitial fluid is the second swellable microprotrusion array, and the method further comprises
applying a first swellable microprotrusion array to a region of skin to absorb interstitial fluid prior to exposure to the stimulus, wherein the region of skin is the same region of skin to which the stimulus is exposed or a different region of skin in the same subject;
removing the microprotrusion array;
recovering miRNA from the microprotrusion array; and
analyzing the miRNA.

In some embodiments, the method comprises:
applying a first swellable microprotrusion array to a region of the skin of a subject to absorb interstitial fluid;
removing the first microprotrusion array;
recovering miRNA from the first microprotrusion array;
analyzing the miRNA from the first microprotrusion array;
exposing a region of skin which is either the same region of skin or a different region in the same subject to a stimulus;
applying a second swellable microprotrusion array to the region of skin exposed to the stimulus to absorb interstitial fluid;
removing the second microprotrusion array;
recovering miRNA from the second microprotrusion array; and
analyzing the miRNA from the second microprotrusion array.

In one or more embodiments, the stimulus comprises a composition. In some embodiments, the composition comprises one or more dermatological active ingredients. In one or more embodiments, exposure to the stimulus comprises topically applying the composition to the region of skin. In some embodiments, the composition is in the form of a solution, suspension, lotion, cream, serum, gel, stick, spray, ointment, liquid wash, soap bar, shampoo, hair conditioner, paste, foam, powder, mousse, shaving cream, hydrogel, or film-forming product. In one or more embodiments, the miRNA recovered from the microprotrusion array applied before administration of the composition is compared to the miRNA recovered from the microprotrusion array applied after administration of the composition. In some embodiments, the composition is administered to a region of skin which is the same type of skin to which the first swellable microprotrusion array is applied. In one or more embodiments, the stimulus is selected from the group consisting of UV light, pollution, irritants, allergens, temperature change, humidity change, and combinations thereof. In some embodiments, the method further comprises a pretreatment step, wherein prior to application of the first swellable microprotrusion array to the region of the skin, the region of skin is exposed to a pretreatment stimulus. In one or more embodiments, the method further comprises:
exposing a region of skin which is either the same region of skin or a different region in the same subject to a second stimulus;
applying a third swellable microprotrusion array to the region of skin exposed to the second stimulus to absorb interstitial fluid;
removing the third microprotrusion array;
recovering miRNA from the third microprotrusion array; and analyzing the miRNA from the third microprotrusion array.

These and other features and advantages of the present invention will be readily apparent from the following detailed description of the invention.

### BRIEF DESCRIPTION OF THE FIGURES

FIGS. 1A-B show a microneedle patch alone and its placement in skin, respectively;
FIG. 2 is a graphical representation showing the distribution of samples on the two first dimensions of a principal component (PC) analysis prior to normalization;
FIG. 3 is a graphical representation showing the signal distribution and total number of reads (inc. median and standard deviation) prior to normalization;
FIG. 4 is a graphical representation the distribution of samples on the two first dimensions of a principal component analysis after normalization;
FIG. 5 is a graphical representation showing the signal distribution and total number of reads (inc. median and standard deviation) after normalization;
FIG. 6 is a dot plot graph showing expression level for skin-enriched miRNA in skin biopsies from the miRNA ATLAS and from the skin samples; and
FIG. 7 is a volcano plot depicting the results of the differential analysis evaluating the impact of retinol topic treatment.

### DETAILED DESCRIPTION OF THE INVENTION

Provided herein are methods relating to the analysis of miRNA in skin by using a microprotrusion array. The methods can provide quantitative data regarding miRNA. When measurements are taken before and after exposure of the skin to some sort of stimulus (*i.e.* a composition), then these methods can also be used to shed light on the epigenetic changes as a result of the exposure to the stimulus. These methods have the benefit of being non-invasive, as they do not involve the removal of tissue, but rather the use of swellable microprotrusion arrays which can be applied to the skin, absorb skin interstitial fluid. The absorbed interstitial fluid contains biomarkers *(i.e.,* miRNAs), which are adsorbed to the microprotrusion array surface. The biomarkers are then extracted from the microprotrusion array through a wash out and samples are analyzed for detection and quantification of the biomarkers of interest.

Accordingly, a first aspect of the invention pertains to a method of quantifying miRNA in skin, the method comprising:
applying a swellable microprotrusion array to a region of skin to absorb interstitial fluid;
removing the microprotrusion array;
recovering miRNA from the interstitial fluid absorbed into the microprotrusion array; and
quantifying the miRNA.

### Swellable Microprotrusion Array

The swellable microprotrusion array comprises a plurality of microprotrusions composed of a swellable polymer composition. As used herein, the term "swellable" means that the polymer composition is able to absorb fluids and increases in mass, and in particular interstitial fluid. In one or more embodiments, the swellable polymer composition is able to absorb sufficient interstitial fluid that it increases by mass by at least 10%, 15%, 20%, 25%, 30% or 35%. In some embodiments, the swellable polymer composition is in its dry state hard and brittle sufficient to penetrate the stratum corneum of mammalian skin. The plurality of microprotrustions may be affixed to a base. The microprotrustions affixed to a base may also be referred to as a "patch". Thus, for example, where the microprotrusions comprise microneedles, the microprotrusion array may be referred to as a "microneedle patch." In preferred embodiments, the swellable microprotrusion array is a microneedle patch. As the swellable microprotrusion array is intended to absorb interstitial fluids for later analysis, it preferably is not impregnated with or does not otherwise have any substance (*e.g*., a drug substance) which could be delivered.

Examples of swellable microprotrusion arrays suitable for use in the methods described herein are described in US Patent No. 9,549,746, the entire contents of which are hereby incorporated by reference.

Microprotrusions can be fabricated from any suitable swellable polymer, which in its dry state is hard and brittle to allow penetration of the stratum corneum, but then which, upon taking up moisture swells to allow absorption of interstitial fluid. Exemplary materials the microprotrusions may be composed of include one or more of a number of polymers known to form hydrogels. Examples of such polymers include, but are not limited to, poly(vinyl alcohol), poly(hydroxyethylmethacrylate), poly(methylvinylether/maleic acid), poly(acrylic acid), poly(caprolactone). In one or more embodiments, the polymers of the microprotrusions are crosslinked, either physically, chemically or both.

A microprotrusion can be any suitable size and shape for use in an array to puncture the stratum corneum. The microprotrusions of the array of the first aspect of the present invention are designed to pierce and optionally cross the stratum corneum. Suitably, the height of the microprotrusions can be altered so as to allow penetration into the upper epidermis, as far as the deep epidermis or even the upper dermis, but not allowing penetration deep enough into the skin to cause bleeding. In one embodiment, the microprotrusions are conical in shape with a circular base which tapers to a point at a height of the microprotrusion above the base.

In embodiments of the microprotrusion array the microprotrusions can be in the range of 1 µm to 3000 µm in height. For example, the microprotrusions can have heights in the range 50 µm to 400 µm, for example 50 to 100 µm. Suitably, in embodiments of the arrays of the invention, microprotrusions can have a width, e.g. diamater in the case of microprotrusions of circular cross-section diameter of 1-500 µm at their base. In one embodiment microprotrusions of and for use in the invention can have a diameter in the range 50-300 µm, for example 100-200 µm. In another embodiment, the microprotrusion of the invention may be of a diameter in the range of 1 µm to 50 µm, for example in the range 20-50 µm.

The apical separation distance between each of the individual microprotusions in an array can be modified to ensure penetration of the skin while having a sufficiently small separation distance to provide high transdermal transport rates. In embodiments of the device the range of apical separation distances between microprotrusions can be in the in the range 50-1000 µm, such as 100-300 µm, for example 100-200 µm. This allows a compromise to be achieved between efficient penetration of the stratum corneum and passage of interstitial fluid.

It will be apparent to those skilled in the art that the microprotrusions of the invention can take any reasonable shape, including, but not limited to, microneedles, cones, rods and/or pillars. As such, the microprotrusions may have the same diameter at the tip as at the base or may taper in diameter in the direction base to tip. The microprotrusions may have at least one sharp edge and may be sharp at the tips. The microprotrusions may be solid, have a hollow bore down at least one longitudinal axis at an angle to the base element and extending to the first side of the base element, they may be porous, or may have at least one channel running down at least one outer surface from tip to base element.

### Methods of Manufacture

Microprotrusions composed of polymers known to form hydrogels can be manufactured by any such methods known in the art. For example, they can be prepared by a micromoulding technique using a master template, such as a microprotrusion array made from one or more of a wide variety of materials, including for example, but not limited to; silicon, metal polymeric material. Master templates can be prepared by a number of methods, including, but not limited to, electrochemical etching, deep plasma etching of silicon, electroplating, wet etch processes, micromoulding, microembossing, "thread-forming" methods and by the use of repetitive sequential deposition and selective x-ray irradiation of radiosensitive polymers to yield solid microprotrusion arrays.

Micromoulds can be prepared by coating the master template with a liquid monomer or polymer which is then cured and the master template removed to leave a mould containing the detail of the master template. In the micromoulding technique, a liquid monomer, with or without initiator and/or crosslinking agent is placed in the mould, which is filled by means of gravitational flow, application of vacuum or centrifugal forces, by application of pressure or by injection moulding. The monomer may then be cured in the mould by means of heat or application of irradiation (for example, light, UV radiation, x-rays) and the formed microprotrusion array, which is an exact replicate of the master template is removed. Alternatively, a solution of a polymer with or without crosslinking agent can be placed in the mould, which is filled by means of gravitational flow, application of vacuum or centrifugal forces, by application of pressure or by injection moulding. The solvent can then be evaporated to leave behind a dried microprotrusion array, which is an exact replicate of the master template, and can then be removed from the mould. The solvents that can be used include, but are not limited to, water, acetone, dichloromethane, ether, diethylether, ethyl acetate. Other suitable solvents will be obvious to one skilled in the art. Micromoulds can also be produced without the need for master templates by, for example, micromachining methods and also other methods that will be obvious to those skilled in the art.

For example, in one embodiment, the microprotrusion arrays may be prepared using micromoulds prepared using a method in which the shape of the desired microprotrusions are drilled into a suitable mould material, for example using a laser and the moulds are then filled using techniques known in the art or as described herein. Microprotrusions composed of polymers known to form hydrogels can also be manufactured using a "self-moulding" method. In this method, the polymeric material is first made into a thin film using techniques well known in the art, including for example, but not limited to, casting, extrusion and moulding. The material may, or may not be crosslinked before the "self moulding" process. In this process, the thin film is placed on a previously-prepared microprotrusion array and heated. Plastic deformation due to gravity causes the polymeric film to deform and, upon hardening, create the desired microprojection structure.

Microprotrusions with a hollow bore can be manufactured by using moulds prepared from hollow master templates or suitably altering the micromachining methods or other methods used to prepare solid microprotrusions. Hollow bores can also be drilled mechanically or by laser into formed microprotrusions. Microprotrusions which have at least one channel running down at least one outer surface from tip to base element can also be produced by suitable modification of the method used to prepare solid microprotrusions. Such alterations will be obvious to those skilled in the art. Channels can also be drilled mechanically or by laser into formed microprotrusions.

Microprotrusions composed of polymers known to form hydrogels can also be manufactured using a "thread forming" method whereby a polymer solution spread on a flat surface has its surface contacted by a projection which is then moved upwards quickly forming a series of polymer "threads", which then dry to form microprotrusions.

In all of the above methods, substances to be incorporated into the microprotrusions themselves (e.g., active therapeutic agents, pore forming agents, enzymes etc.) can be added into the liquid monomer or polymer solution during the manufacturing process. Alternatively, such substances can be imbibed from their solution state in a solution used to swell the formed microprotrusion arrays and dried thereafter or the formed arrays can be dipped into a solution containing the agent of interest or sprayed with a solution containing the agent of interest. Solvents used to make these solutions include water, acetone, dichloromethane, ether, diethylether, ethyl acetate. Other suitable solvents will be obvious to those skilled in the art, as will the processes used to dry the microprotrusion arrays. If the microprotrusions and/or base elements are to be made adhesive, the formed arrays can be dipped into a solution containing an adhesive agent or sprayed with a solution containing an adhesive agent. The adhesive agents used can be a pressure sensitive adhesive or a bioadhesive. These substances are well known and will be readily available to those skilled in the art.

The base element on which the microprotrusions are formed can be varied in thickness by suitable modification of the method of manufacture, including, for example, but not limited to increasing the quantity of liquid monomer or polymer solution used in the manufacturing process. In this way the barrier to diffusion/transport of therapeutic active agents and/or analytes of interest can be controlled so as to achieve, for example rapid delivery or sampling or sustained release. Where therapeutic active agent(s) is/are to be contained within the matrix of the microprotrusions and base element, the thickness of the base element can usefully be increased so as it functions as a fully integrated reservoir.

### Application and Removal of the Swellable Microprotrusion Array

The swellable microprotrusion array may be applied to skin by any suitable method which will result penetrate the stratum corneum of mammalian skin and allow absorption of interstitial fluid. The swellable microprotrusion array may be applied using a force with sufficient force to penetrate the stratum corneum but not otherwise damage the swellable microprotrusion array. An applicator may be used to assist with application of the swellable microprotrusion array. The pressure may be applied for any period sufficient to ensure penetration into the skin. For example, pressure may be applied from about 1, 2, 3, 4, 5, 10, 15, 20, 25, 30, 40, 50 or 60 seconds to about 5, 10, 15, 20, 25, 30, 40, 50 or 60 seconds. The swellable microprotrusion array may then be further secured in place, for example, by affixing an adhesive tape over the swellable microprotrusion array and skin.

The swellable microprotrusion array may be applied to a region on any type of skin on the body, for example skin of the face, lips, neck, chest, back, buttocks, arms, axilla, and/or legs. In one or more embodiments, the skin is from a live subject. In some embodiments, the skin is an *ex vivo* sample).

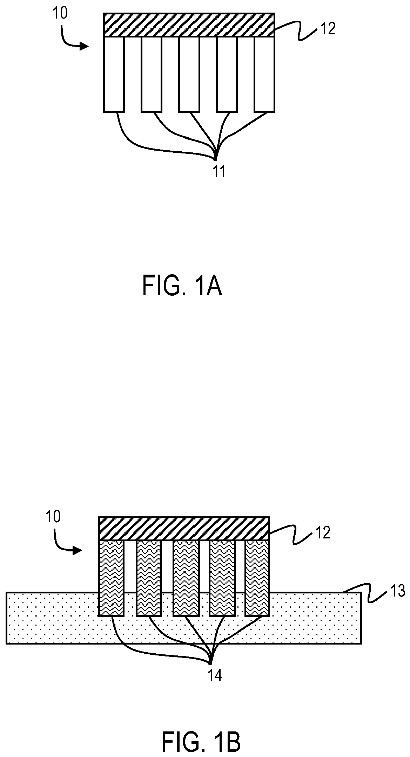

FIGS. 1A-1B are diagrams showing a swellable microprotrusion array 10 in accordance with one or more embodiments of the invention alone (FIG. 1A) and when placed in skin 13 (FIG. 1B). As seen in FIG. 1A, the swellable microprotrusion array 10 may contain several microprotrusions 11 affixed on a base 12. In FIG. 1B, swellable microprotrusion array 10 has been inserted into skin 13. As seen in the figure, the microprotrusions 14 swell after absorbing interstitial fluid from the skin 13.

Once applied, the swellable microprotrusion array is left on the skin long enough to swell and absorb enough interstitial fluid for analysis of miRNA. In one or more embodiments, the swellable microprotrusion array is left on the skin for at least about 5, 10, 15, 20, 25 or 30 minutes to about 30, 60, 90, 120, 150 or 180 minutes. In some embodiments, the swellable microprotrusion array is left on the skin for at least about 20 to about 180 minutes. In further embodiments, the swellable microprotrusion array is left on the skin for at least about 30 to about 120 minutes. After sufficient time, the swellable microprotrusion array may then be removed from the skin.

### Recovery of miRNA From the Interstitial Fluid

Once the swellable microprotrusion array has been removed from the skin, the miRNA is recovered from the interstitial fluid. This may be done by washing the swellable microprotrusion array, which can wash off at least a portion of the absorbed interstitial fluid to recover the miRNA from. The swellable microprotrusion array may be washed with a buffer selected from the group consisting of distilled water, phosphate-buffered saline, and an RNA stabilization reagent (such as the PAXgene^{®} Blood RNA Tube), to provide a washing liquid. The washing liquid contains at least a portion of the interstitial fluid (and thus miRNA). The washing liquid may then be filtrated and/or concentrated further.

### Quantification of the miRNA

Quantifying the miRNA may be accomplished through any methods known in the art. In some embodiments, quantification of the miRNA comprises next generation sequencing, transcriptome analysis, or quantitative polymerase chain reaction. Quantification of the miRNA may also be accomplished using other transcriptome analysis like microarray, utilization of PCR, QuantiGene^{™} Singleplex Assay Kit from Thermofisher.

After quantification of the miRNA, the method may further comprise comparing the quantified miRNA to a reference. The reference may comprise any source of miRNA information to compare the quantified miRNA to. Examples include, but are not limited to literature sources, and historical miRNA data for the source of the skin sample. In some embodiments, the quantified miRNA is compared to quantified miRNA from the same subject and/or the same type of skin from a previous point in time (for example, before exposure to a stimulus).

The reference may be from a different type of skin or from a different subject, as the case may call for and depending on the comparisons being made. For example, skin which is deemed to be healthy may be compared to skin deemed to be in a disease state or having some other condition (*e.g*., dry skin, dark spots (solar lentigo), acne, dermatitis, psoriasis, eczema, rosacea, vitiligo, keloids, melanoma, other skin cancers, acne *etc*.), and could either come from different types of skin on the same subject or different subjects. In embodiments on the same subject, one sample could be taken from an affected region of skin and compared to a non-affected region of skin. In another example, skin from one type of skin may be compared to another (*i.e*., skin from one body part is compared to the skin from another body part on the same subject). Thus, in one embodiment, skin from face could be compared to the body, or skin from one body part could be compared to another body part. In another example, skin from one subject in a first age group (*e.g*., young or baby skin) may be compared to skin from another subject in a second age group (*e.g*., adult). In yet another example, skin from one ethnic group or phototype could be compared to the skin from another ethnic group or phototype. Such comparisons can be used to create general trends of miRNA expression across the population.

### Monitoring Epigenetic Changes

As described above, swellable microprotrusion array methods described herein allow for monitoring epigenetic changes in skin as a result of a stimulus. Such stimuli incudes, but not limited to, UV light, pollution, irritants, allergens, temperature change, humidity change, and combinations thereof. A stimulus of particular interest is in the area of skin care compositions (*e.g*., cosmetic, pharmacological or other dermatologically acceptable compositions).

Accordingly, another aspect of the invention pertains to a method of monitoring epigenetic changes in skin as a result of exposure to a stimulus, the method comprising:
exposing a region of the skin of a subject to the stimulus;
applying a swellable microprotrusion array to at least a part of the region of skin to absorb interstitial fluid;
removing the microprotrusion array;
recovering miRNA from the microprotrusion array; and
analyzing the miRNA.

The steps of application of the swellable microprotrusion array, removal of the swellable microprotrusion array, and recovery of miRNA may all be carried out as described previously. Analysis of miRNA may comprise quantification of the miRNA as described above, but may also comprise any other known methods useful in the analysis of epigenetic changes.

As discussed above, after quantification of the miRNA, the quantified miRNA is compared to quantified miRNA from the same subject and/or the same type of skin from a previous point in time (for example, before exposure to a stimulus). In one or more embodiments, the analyzed miRNA is compared to miRNA is also obtained using a swellable microprotrusion array prior to exposure to a stimulus (*e.g*., administration of a composition). Thus, in some embodiments, the swellable microprotrusion array that is applied to at least a part of the region of skin to absorb interstitial fluid is the second swellable microprotrusion array, and the method further comprises
applying a first swellable microprotrusion array to a region of skin to absorb interstitial fluid prior to administration of the composition, wherein the region of skin is the same region of skin to which the composition is applied or a different region of skin in the same subject;
removing the microprotrusion array;
recovering miRNA from the microprotrusion array; and
analyzing the miRNA.

In some embodiments, the miRNA recovered from the microprotrusion array applied before exposure to the stimulus (*e.g*., administration of a composition) is compared to the miRNA recovered from the microprotrusion array applied after exposure to the stimulus (*e.g*., administration of the composition).

Thus another aspect of the invention pertains to a method of monitoring epigenetic changes in skin as a result of exposure to a stimulus, the method comprising:
applying a first swellable microprotrusion array to a region of the skin of a subject to absorb interstitial fluid;
removing the first microprotrusion array;
recovering miRNA from the first microprotrusion array;
analyzing the miRNA from the first microprotrusion array;
exposing a region of skin which is either the same region of skin or a different region in the same subject to a stimulus;
applying a second swellable microprotrusion array to the region of skin to exposed to the stimulus to absorb interstitial fluid;
removing the second microprotrusion array;
recovering miRNA from the second microprotrusion array; and
analyzing the miRNA from the second microprotrusion array.

In some embodiments, the miRNA recovered from the microprotrusion array applied before administration of the composition is compared to the miRNA recovered from the microprotrusion array applied after administration of the composition.

In embodiments where the epigenetic effect of exposure to a stimulus (*e.g*., administration of a composition) is being analyzed, the first and second swellable microprotrusion arrays and compositions are all preferably used on the same type of skin. As used herein, "type of skin" refers to skin that is found on the same anatomical part of the body. For example, if a first sample is taken from arm skin, then the composition and second sample are preferably also taken from arm skin. However, this does not mean that the exact same region of skin is used for the first and second swellable microprotrusion arrays and compositions. That is, the composition and second swellable microprotrusion array do not need to be used on the exact same region that the first swellable microprotrusion array was used on. In some embodiments, the composition is administered to a region of skin which is the same type of skin to which the first swellable microprotrusion array is applied.

In some instances, it may be desired to incorporate multiple stimuli. In some embodiments, the method may further comprise a pretreatment step, wherein prior to application of the first swellable microprotrusion array to the region of the skin, the region of skin is exposed to a pretreatment stimulus. This provides an initial condition in the skin, and then the effect of a second stimuli can be analyzed. For example, the pretreatment step may comprise exposure of the skin to a UV sun filter, and then the second stimulus could comprise exposure to UV light. The epigenetic changes could then shed light on the effect of the UV filter in preventing damage from the UV light.

In some embodiments, the method may further comprise
exposing a region of skin which is either the same region of skin or a different region in the same subj ect to a second stimulus;
applying a third swellable microprotrusion array to the region of skin exposed to the second stimulus to absorb interstitial fluid;
removing the third microprotrusion array;
recovering miRNA from the third microprotrusion array; and
analyzing the miRNA from the third microprotrusion array.

Depending on the number of stimuli desired, the process can be repeated to follow the effects of the various stimuli.

### Administration of a Composition

Any suitable method of applying a composition to the skin may be used. In some embodiments, administration of the composition comprises topically applying the composition to the region of skin. For example, the composition may be applied directly from a package to the skin, by hand to the skin, or may be transferred from a substrate such as a wipe or mask, or a combination of two or more thereof. In one or more embodiments, the composition may be applied via a dropper, tube, roller, spray, and patch or added to a bath or otherwise to water to be applied to the skin, and the like. In one or more embodiments, the composition is in the form of a solution, suspension, emulsion, lotion, cream, serum, gel, stick, spray, ointment, liquid wash, soap bar, shampoo, hair conditioner, paste, foam, powder, mousse, shaving cream, hydrogel, or film-forming product.

Such topical application may be to any skin on the body, for example skin of the face, lips, neck, chest, back, buttocks, arms, axilla, and/or legs.

### Compositions

A wide variety of compositions may be used in the methods described herein. Such compositions may include a single ingredient to evaluate the epigenetic changes due to the single ingredient. Alternatively, the compositions may comprise a multitude of ingredients to evaluate the epigenetic changes resulting from exposure of the skin to multiple ingredients at once. Compositions may include any compound or compounds, the effect of which is of interest. Examples include, but are not limited to, dermatological active ingredients. The compositions may come in any form including, but not limited to, single ingredients delivered in a carrier, lotions, creams, ointments, and emulsions.

The compositions of the present invention can also be formulated into a solid formulation (e.g., a wax-based stick, soap bar composition, powder, or wipe). The composition of the present invention can also be combined with a solid, semi-solid, or dissolvable substrate (*e.g*., a wipe, mask, pad, glove, or strip).

### Dermatological Active Ingredients

The compositions of the present invention may further comprise any of a variety of dermatological active ingredients. As used herein, "dermatological active agent" refers to an ingredient has an effect on the skin. Examples of dermatological active ingredients agents include: skin lightening agents, darkening agents, additional anti-aging agents, tropoelastin promoters, collagen promoters, anti-acne agents, shine control agents, anti-microbial agents such as anti-yeast agents, anti-fungal, and anti-bacterial agents, anti-inflammatory agents, anti-parasite agents, external analgesics, sunscreens, photoprotectors, antioxidants, keratolytic agents, detergents/surfactants, moisturizers, nutrients, vitamins, energy enhancers, anti-perspiration agents, astringents, deodorants, hair removers, hair growth enhancing agents, hair growth delaying agents, firming agents, hydration boosters, efficacy boosters, anti-callous agents, agents for skin conditioning, anti-cellulite agents, odor-control agents such as odor masking or pH changing agents, and the like.

Examples of various suitable dermatological active ingredients include hydroxy acids; benzoyl peroxide; D-panthenol; UV filters such as but not limited to avobenzone (Parsol 1789), bisdisulizole disodium (Neo Heliopan AP), diethylamino hydroxybenzoyl hexyl benzoate (Uvinul A Plus), ecamsule (Mexoryl SX), methyl anthranilate, 4-aminobenzoic acid (PABA), cinoxate, ethylhexyl triazone (Uvinul T 150), homosalate, 4-methylbenzylidene camphor (Parsol 5000), octyl methoxycinnamate (Octinoxate), octyl salicylate (Octisalate), padimate O (Escalol 507), phenylbenzimidazole sulfonic acid (Ensulizole), polysilicone-15 (Parsol SLX), trolamine salicylate, Bemotrizinol (Tinosorb S), benzophenones 1-12, dioxybenzone, drometrizole trisiloxane (Mexoryl XL), iscotrizinol (Uvasorb HEB), octocrylene, oxybenzone (Eusolex 4360), sulisobenzone, bisoctrizole (Tinosorb M), titanium dioxide, zinc oxide; carotenoids; free radical scavengers; spin traps; retinoids and retinoid precursors such as retinol, retinoic acid and retinyl palmitate; ceramides; polyunsaturated fatty acids; essential fatty acids; enzymes; enzyme inhibitors; minerals; hormones such as estrogens; steroids such as hydrocortisone; 2-dimethylaminoethanol; copper salts such as copper chloride; peptides containing copper such as Cu:Gly-His-Lys, coenzyme Q10; amino acids such a proline; vitamins; lactobionic acid; acetyl-coenzyme A; niacin; riboflavin; thiamin; ribose; electron transporters such as NADH and FADH2; and other botanical extracts such as oat, aloe vera, Feverfew, Soy, Shiitake mushroom extracts, and derivatives and mixtures thereof.

If present, any additional active agent (such as any dermatological active ingredient) may be present in a composition in any suitable amount, for example, in an amount of from about 0.0001% to about 20% by weight of the composition, e.g., about 0.001% to about 10% such as about 0.01% to about 5%. In some embodiments, in an amount of 0.1% to 5% and in other embodiments from 1% to 2%.

Compositions of the present invention may include one or more additional anti-inflammatory compounds. Examples of suitable anti-inflammatory agents include substituted resorcinols, (E)-3-(4-methylphenylsulfonyl)-2-propenenitrile (such as "Bay 11-7082," commercially available from Sigma-Aldrich of St. Louis, Missouri), tetrahydrocurcuminoids (such as Tetrahydrocurcuminoid CG, available from Sabinsa Corporation of Piscataway, NJ), extracts and materials derived from the following: *Phellodendron amurense* Cortex Extract (PCE), Non-Denatured Soy (*Glycine max*), Feverfew (*Tanacetum parthenium*), Ginger (*Zingiber officinale*), Ginkgo (*Ginkgo biloba*), Madecassoside (*Centella asiatica* extract ingredient), Cotinus (*Cotinus coggygria*), Butterbur Extract (*Petasites hybridus*), Goji Berry (*Lycium barbarum*), Milk Thistle Extract (*Silybum marianum*), Honeysuckle (*Lonicera japonica*), Basalm of Peru (*Myroxylon pereirae*), Sage (*Salvia officinalis*), Cranberry Extract (*Vaccinium oxycoccos*), Amaranth Oil *(Amaranthus cruentus*), Pomegranate (*Punica granatum*), Yerbe Mate (*Ilex paraguariensis* Leaf Extract), White Lily Flower Extract (*Lilium candidum*), Olive Leaf Extract (*Olea europaea*), Phloretin (apple extract), Oat Flour (*Aveena sativa*), Lifenol (Hops: *Humulus lupulus*) Extract, Bugrane P (*Ononis spinosa*), Licochalcone (Licorice: *Glycyrrhiza inflate* extract ingredient), Symrelief (Bisabolol and Ginger extract), combinations of two or more thereof, and the like.

One example of an anti-inflammatory agent is a resorcinol. Particularly suitable substituted resorcinols include 4-hexyl resorcinol and 4-octylresorcinol, particularly 4-hexyl resorcinol. 4-Hexyl resorcinol is commercially available as "SYNOVEA HR" from Sytheon of Lincoln Park, NJ. 4-Octylresorcinol is commercially available from City Chemical LLC of West Haven, Connecticut.

By "extracts of feverfew," it is meant extracts of the plant "*Tanacetum parthenium,"* such as may be produced according to the details set for the in US Patent No. 7,537,791, entitled "PARTHENOLIDE FREE BIOACTIVE INGREDIENTS FROM FEVERFEW (TANACETUM PARTHENIUM) AND PROCESSES FOR THEIR PRODUCTION." One particularly suitable feverfew extract is commercially available as about 20% active feverfew, from Integrated Botanical Technologies of Ossining, NY.

A variety of other materials may also be present in the compositions of the present invention. In one or more embodiments, the composition comprises one or more topical ingredients selected from the group consisting of: surfactants, chelating agents, emollients, humectants, conditioners, preservatives, opacifiers, fragrances and the like.

What is meant by an emollient is a compound that helps to maintain the soft, smooth, and pliable appearance of the skin (*e.g*., by remaining on the skin surface or in the stratum corneum to act as a lubricant). Examples of suitable emollients include those found in Chapter 35, pages 399-415 (Skin Feel Agents, by G Zocchi) in Handbook of Cosmetic Science and Technology (edited by A. Barel, M. Paye and H. Maibach, Published in 2001 by Marcel Dekker, Inc New York, NY), and include, but are not limited to, petrolatum, hexyldecyl stearate and plant, nut, and vegetable oils such as macadamia nut oil, rice bran oil, grape seed oil, palm oil, prim rose oil, hydrogenates peanut oil, and avocado oil.

What is meant by a humectant is a compound intended to increase the water content of the top layers of skin (*e.g*., hygroscopic compounds). Examples of suitable humectants include those found in Chapter 35, pages 399-415 (Skin Feel Agents, by G Zocchi) in Handbook of Cosmetic Science and Technology (edited by A. Barel, M. Paye and H. Maibach, Published in 2001 by Marcel Dekker, Inc New York, NY) and include, but are not limited to, glycerin, sorbitol or trehalose (*e.g*., α,α- trehalose, β,β-trehalose, α,β-trehalose) or a salt or ester thereof (*e.g*., trehalose 6-phosphate).

What is meant by a surfactant is a surface-active agent intended to cleanse or emulsify. Examples of suitable surfactants include those found in Chapter 37, pages 431-450 (Classification of surfactants, by L. Oldenhove de Guertechin) in Handbook of Cosmetic Science and Technology (edited by A. Barel, M. Paye and H. Maibach, Published in 2001 by Marcel Dekker, Inc., New York, NY) and include, but are not limited to anionic surfactants such as sulfates and sodium coco glycinate, cationic surfactants, amphoteric surfactants such as betaines, nonionic surfactants such as alkyl polyglucosides. Examples of suitable chelating agents include those which are capable of protecting and preserving the compositions of this invention. In one or more embodiments, the chelating agent is ethylenediaminetetraacetic acid ("EDTA"), and more specifically is tetrasodium EDTA, available commercially from Dow Chemical Company of Midland, Michigan under the trade name, "Versene 100XL."

Suitable preservatives include, for example, parabens, quaternary ammonium species, phenoxyethanol, benzoates, DMDM hydantoin, organic acids and are present in the composition in an amount, based upon the total weight of the composition, from about 0 to about 1 percent or from about 0.05 percent to about 0.5 percent.

Examples of conditioners include, but are not limited to, volatile silicone conditioning agent having an atmospheric pressure boiling point less than about 220°C. Examples of suitable volatile silicones nonexclusively include polydimethylsiloxane, polydimethylcyclosiloxane, hexamethyldisiloxane, cyclomethicone fluids such as polydimethylcyclosiloxane available commercially from Dow Corning Corporation of Midland, Michigan under the tradename, "DC-345" and mixtures thereof, and specifically include cyclomethicone fluids. Other suitable conditioners include cationic polymers, including polyquarterniums, cationic guar, and the like.

Any suitable carrier may be used in the compositions as the context dictates. In some embodiments, the carrier is a dermatologically acceptable carrier. As will be recognized by those of skill in the art, dermatologically acceptable carriers comprise carriers that are suitable for use in contact with the body, in particular the skin, without undue toxicity, incompatibility, instability, irritation, allergic response, and the like. A safe and effective amount of dermatologically carrier is from about 50, 55, 60, 65, 70, 75, 80, 85, 90, 95, or 98% to about 85, 90, 95, 98, 99, 99.1, 99.5 or 99.9% by weight of the composition.

The following are non-limiting examples of dermatologically acceptable carriers. Other dermatologically acceptable carriers can be formulated by those of ordinary skill in the art. In one embodiment, the dermatologically carrier contains water. In a further embodiment, the dermatologically acceptable carrier may also contain one or more aqueous or organic solvents. Examples of organic solvents include, but are not limited to: dimethyl isosorbide; isopropyl myristate; surfactants of cationic, anionic and nonionic nature; vegetable oils; mineral oils; waxes; gums; synthetic and natural gelling agents; alkanols; glycols; and polyols. Examples of glycols include, but are not limited to, glycerin, propylene glycol, butylene glycol, pentalene glycol, hexylene glycol, polyethylene glycol, polypropylene glycol, diethylene glycol, triethylene glycol, capryl glycol, glycerol, butanediol and hexanetriol, and copolymers or mixtures thereof. Examples of alkanols include, but are not limited to, those having from about 2 carbon atoms to about 12 carbon atoms (*e.g*., from about 2 carbon atoms to about 4 carbon atoms), such as isopropanol and ethanol. Examples of polyols include, but are not limited to, those having from about 2 carbon atoms to about 15 carbon atoms (*e.g*., from about 2 carbon atoms to about 10 carbon atoms) such as propylene glycol. The organic solvents may be present in the carrier in an amount, based upon the total weight of the carrier, of from about 1 percent to about 99.99 percent (*e.g*., from about 20 percent to about 50 percent). Water may be present in the carrier (prior to use) in an amount, based upon the total weight of the carrier, of from about 5 percent to about 95 percent (e.g., from about 50 percent to about 90 percent). Solutions may contain any suitable amounts of solvent, including from about 40 to about 99.99%. Some solutions contain from about 50 to about 99.9%, from about 60 to about 99%, from about 70 to about 99%, from about 80 to about 99%, or from about 90 to 99% of solvent. Single emulsion skin care preparations, such as lotions and creams, of the oil-in-water type, and water-in-oil type are well-known in the art and are useful in the subject invention. Multiphase emulsion compositions, such as the water-in-oil-in-water type or the oil-in-water-in-oil type, are also useful in the subject invention. In general, such single or multiphase emulsions contain water, emollients, and emulsifiers as essential ingredients. The compositions of this invention can also be formulated as a gel (*e.g*., an aqueous, alcohol, alcohol/water, or oil gel using a suitable gelling agent(s)). Suitable gelling agents for aqueous and/or alcoholic gels include, but are not limited to, natural gums, acrylic acid and acrylate polymers, and copolymers, and cellulose derivatives (*e.g*., hydroxymethyl cellulose and hydroxypropyl cellulose). Suitable gelling agents for oils (such as mineral oil) include, but are not limited to, hydrogenated butylene/ethylene/styrene copolymer and hydrogenated ethylene/propylene/styrene copolymer. Such gels typically contains between about 0.1% and 5%, by weight, of such gelling agents.

### Other Stimuli

In addition to the compositions described above, epigenetic changes due to other types of stimuli may be evaluated using the methods described herein. This includes, but not limited to, a changes in the environment or exposure to other non-composition stimuli. Examples of environmental variables include, but are not limited to temperature, humidity, air pollution, irritants, *etc..* Other non-composition stimuli include, but are not limited to, types of light or radiation (*e.g*., UV light).

While the foregoing description represent exemplary embodiments of the present invention, it will be understood that various additions, modifications and substitutions may be made therein without departing from the spirit and scope of the present invention. In particular, it will be clear to those skilled in the art that the present invention may be embodied in other specific forms, structures, arrangements, proportions, and with other elements, materials, and components, without departing from the spirit or essential characteristics thereof. One skilled in the art will appreciate that the invention may be used with many modifications of structure, arrangement, proportions, materials, and components and otherwise, used in the practice of the invention, which are particularly adapted to specific environments and operative requirements without departing from the principles of the present invention. The presently disclosed embodiments are therefore to be considered in all respects as illustrative and not restrictive, the scope of the invention being indicated by the appended claims, and not limited to the foregoing description. It will be appreciated that in the claims, the term "comprises/comprising" does not exclude the presence of other elements or steps. In addition, singular references do not exclude a plurality. The terms "a", "an", "first", "second", etc., do not preclude a plurality.

All percentages, parts and ratios are based upon the total weight of the composition of the present invention, unless otherwise specified. All such weights as they pertain to the listed ingredients are based on the level of the particular ingredient described and, therefore, do not include carriers or by-products that may be included in commercially available materials, unless otherwise specified.

As used herein, "essentially free" or "substantially free" of an ingredient means containing less than 0.1 weight percent, or less than 0.01 weight percent, or none of an ingredient.

To provide a more concise description, some of the quantitative expressions given herein are not qualified with the term "about". It is understood that whether the term "about" is used explicitly or not, every quantity given herein is meant to refer to the actual given value, and it is also meant to refer to the approximation to such given value that would reasonably be inferred based on the ordinary skill in the art, including approximations due to the experimental and/or measurement conditions for such given value.

To provide a more concise description, some of the quantitative expressions herein are recited as a range from about amount X to about amount Y. It is understood that wherein a range is recited, the range is not limited to the recited upper and lower bounds, but rather includes the full range from about amount X through about amount Y, or any amount or range therein.

### EXAMPLES

### EXAMPLE 1 - Validation of the Swelling Capacities of the Microneedle Patch in the Skin

Several microneedle patches were weighed before and after insertion on *ex vivo* human skin explants to evaluate the swelling capacities of the microneedle patch in the skin. The microneedle patch comprised 361 (19 x 19) needles, perpendicular to the base and of pyramidal shape and height 600 µm, with base width 300 µm and needle interspacing 150 µm on a 0.5 cm² area. A composition containing the material shown below in Table 1 was cast into a microneedle mold.

**Table 1: Micronedle Patch Composition**

| **Material** | **Composition (% w/w)** |
|---|---|
| PVM/MA Copolymer¹ | 20 |
| PEG 10,000² | 7.5 |
| Na₂CO₃ | 3.0 |
| Water | 69.5 |

| | |
|---|---|
| 1. Gantrez^{™} S-97 from Ashland 2. Poly(ethyleneglycol) (PEG; molecular weight 10,000 Da) was purchased from Sigma-Aldrich, Dorset, UK | |

### Materials and methods

Abdominal skin tissue was obtained from normal human adults undergoing plastic surgery. For the *ex vivo* experiment, a piece of skin (25cm²) was cut under sterile conditions and placed on a thick filter paper soaked in culture medium (KGM^{™} Gold^{™} with supplements from Lonza) in a petri dish. Four microneedle patches were weighed and then inserted into skin (Samples MN1-4). The flat end of a plunger from a 5 ml syringe was pressed against the base of the microneedle patch in order to insert them into the skin using firm pressure over 30 seconds. A clear tape was placed on each microneedle patch to fix them into skin. The microneedle patches were maintained in place for 120 min. To mimic *in vivo* conditions, the skin sample was incubated at 37 °C over the course of the experiment. A microneedle patch was placed also in the incubator without being inserted into the skin (Sample MN0). After the incubation, all microneedle patches were again weighed. Then, the microneedle patches were washed with 200µL of phosphate-buffered saline (PBS) IX (Lonza) by inverting tube 4 times. The washing liquids were picked up and measured using the Qubit^{™} miRNA Assay Kit from ThermoFisher Scientific.

### Results

The results are shown below in Table 1.

**Table 1: Weight Measurements**

| Sample | Weight (mg) | | |
|---|---|---|---|
| | Before Incubation | After Incubation | Difference |
| MN0 (Comp.) | 19.2 | 23 | 3.8 |
| MN1 | 28.4 | 43.6 | 11.4^{∗} |
| MN2 | 31.4 | 46.4 | 11.2^{∗} |
| MN3 | 20.3 | 32.1 | 8^{∗} |
| MN4 | 22 | 39.9 | 14.1^{∗} |
| | | Mean | 11.18^{∗} |
| | | SD | 2.50 |

| | | | |
|---|---|---|---|
| ^{∗}The weight of MN without skin (i.e. the difference in weight of Sample MN0) is subtracted from MN into skin (i.e. from the weight of Samples MN1-MN4 after incubation). | | | |

### Conclusion:

As can be seen from the above table, there is an increase of weight for samples MN1-4 after insertion in the skin. This confirms the uptake of biofluids from the skin explant into the microneedle patch (*i.e*., swelling capacities).

### EXAMPLE 2 - Analysis of Sampled Biofluids to Detect and Quantify miRNA Content

Several samples were analyzed using the HTG EdgeSeq miRNA whole transcriptome assay (HTG Molecular Diagnostics, Inc., Tuscson, AZ) to detect miRNAs and to assess various elution/extraction methods to retrieve those miRNAs. A summary of the sample conditions is shown in Table 2 below:

### Conditions

**Table 2: Summary of Samples**

| Sample ID | Sample Description | Patch (Y/N) | Biomaterial | Sonication (Y/N) | Concentration | | Wash/Elution buffer |
|---|---|---|---|---|---|---|---|
| | | | | | Y/N | if Y, Concentrate or Filtrate | |
| Ex. 1 | Skin Digestion Positive Control without microneedle | N | Skin Digestion | N | N | - | - |
| Ex. 2 | Skin Digestion Positive Control with micro needle | Y | Skin Digestion | N | N | - | PBS |
| Ex. 3 | PBS Negative Control | Y | PBS | N | N | - | PBS |
| Ex. 4 | Skin Test sample - 1 | Y | Ex Vivo Skin | N | N | - | PBS |
| Ex. 5 | Skin Test sample - 2 | Y | Ex Vivo Skin | N | N | - | PBS |
| Ex. 6 | Skin Test sample - 3 | Y | Ex Vivo Skin | N | N | - | PBS |
| Ex. 7 | Skin Test sample - 4 | Y | Ex Vivo Skin | N | N | - | PBS |
| Ex. 8 | Skin Test sample Concentrate | Y | Ex Vivo Skin | N | Y | C | PBS |
| Ex. 9 | Skin Test sample Filtrate | Y | Ex Vivo Skin | N | Y | F | PBS |
| Ex. 10 | Skin Test sample Sonicated | Y | Ex Vivo Skin | Y | N | - | PBS |
| Ex. 11 | Skin Test sample Sonicated Concentrate | Y | Ex Vivo Skin | Y | Y | C | PBS |
| Ex. 12 | Skin Test sample Sonicated Filtrate | Y | Ex Vivo Skin | Y | Y | F | PBS |

### Materials & Methods:

### Ex. 1-3:

Abdominal skin explants were obtained from normal human adults undergoing plastic surgery. For the *ex vivo* experiment, four 1.13-cm² explants were punched in the skin samples under sterile conditions. The explants were placed in pairs in a 1.5 ml sterile tube containing 500µL of trypsin 0.05% without phenol red and incubated under a 5% CO₂ humidified atmosphere overnight at 37 °C. The tubes were then vortexed, and the explants were removed from the tube after pressing them with a tip. The liquid was centrifugated at 900g for 5min to remove cells and tissue debris. The supernatants were then collected. 500µl of this skin digestion was analyzed as sample Ex. 1.

An aluminium foil was placed onto wax. Two microneedles were placed in the center of the foil, needles facing downwards, and these were inserted by manual force, using the end of a 5ml syringe plunger through the aluminum foil. The microneedles, now inserted into the foil, were carefully removed from the wax and placed onto the top of the petri dish containing skin digestion (Ex. 2 sample) or 500µl of PBS IX (Ex. 3 sample). After 120 minutes of incubation, the microneedles were removed. Then, the 2 microneedles were washed together with 500µL of PBS IX (Lonza) by inverting tube 4 times and the microneedles were removed.

### Ex. 4-12:

Abdominal skin biopsy was obtained from normal human adults undergoing plastic surgery. For the *ex vivo* experiment, eighteen 1.13-cm² explants were punched into the skin samples under sterile conditions and placed on a thick filter paper soaked in culture medium (KGM^{™} Gold^{™} with supplements from Lonza) in a petri dish. Microneedles were inserted into skin explant. The flat end of a plunger from a 5 ml syringe was pressed against the base of the microneedle patch in order to insert them into the skin explant using firm pressure over 30 seconds. A clear tape was placed on each microneedle patch to fix them into skin explant. The microneedle patches were maintained in place for 120 min. To mimic *in vivo* conditions, the skin explants were incubated at 37 °C over the course of this experiment. After incubation, 2 microneedle patches were washed together with 500µL of PBS IX (Lonza) by inverting tube 4 times. The washing liquids were picked up (except for Ex. 10-12).

For Ex. 10-12, after the incubation, the microneedle patches were washed together with 500µL of PBS IX (Lonza) by inverting tube 4 times and then placed in a sonicator for 15 minutes. Then, the liquids were retrieved from the tube and transferred into a new tube, leaving the microneedle patch behind.

Ex. 8-9 and 11-12 were treated via Amicon^{®} centrifugal filter device to generate filtrate and concentrate samples. Briefly, the washing liquids from Ex.8-9 and 11-12 were filled into Amicon^{®} centrifugal filter tubes. The tubes were then spun with a centrifuge to allow the liquid to pass through the filter. The filtered liquid was then recovered and referred to as "filtrate". The remaining liquid that did not pass through the filter was also recovered and referred to as "concentrate". All samples were sent to HTG Molecular Diagnostics to run an EdgeSeq miRNA whole transcriptome assay.

### Results

All the statistical analyses were performed in the R 4.0.0 environment, although the skilled person would appreciate that other suitable environments are available.

### Data Pre-Processing and Normalization

The data provided by HTG after EdgeSeq miRNA whole transcriptome assay is formatted as a 2102 rows x 27 columns plain-text raw count matrix. Among the 2102 miRNA, five correspond to internal control, thirteen correspond to housekeeping probes, and 2084 to miRNA. Lowly expressed miRNA (*i.e*., with read count lower than the 1st decile in more than two samples) are removed for subsequent analysis to avoid artifacts.

Before diving into detailed analyses, the count matrix was normalized using a combination of quantile normalization and an additional round of normalization relying on housekeeping probes. The objective of this normalization is to make the libraries / samples comparable between each other by:
- scaling the total number of reads per sample to the same value,
- scaling the median and the standard deviation across samples to the same values,
- scaling the count value for housekeeping probes across samples to the same values.

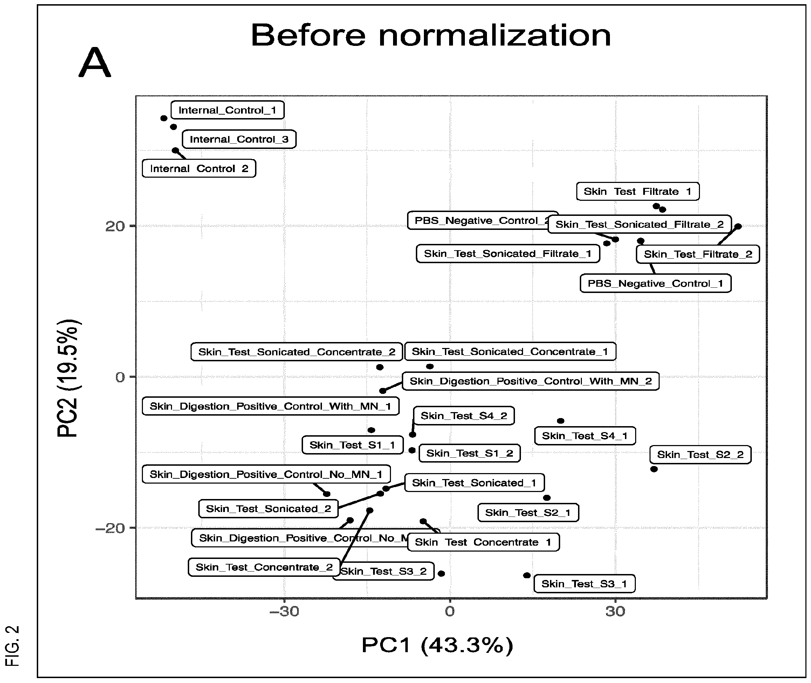

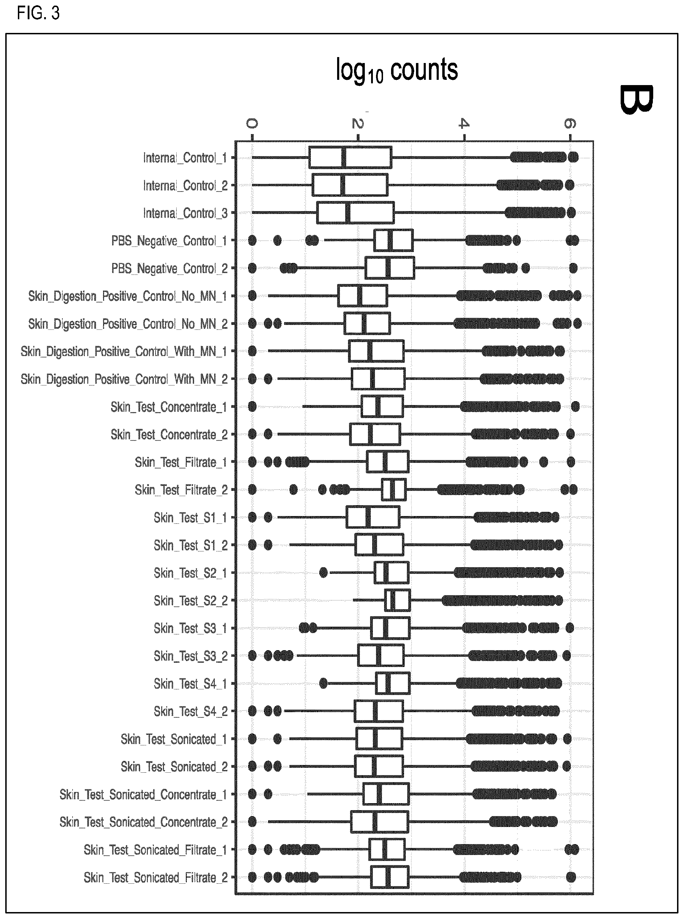

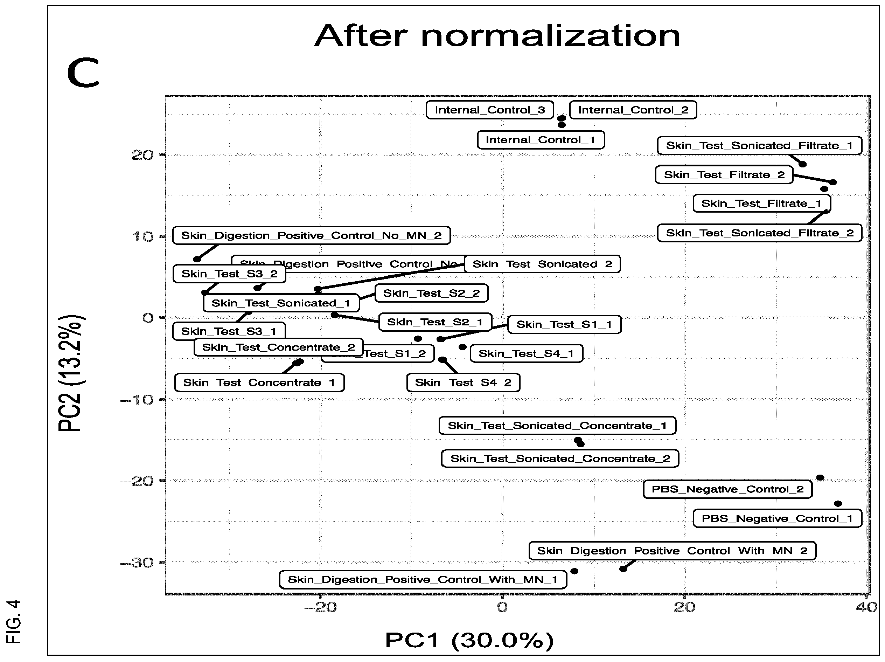

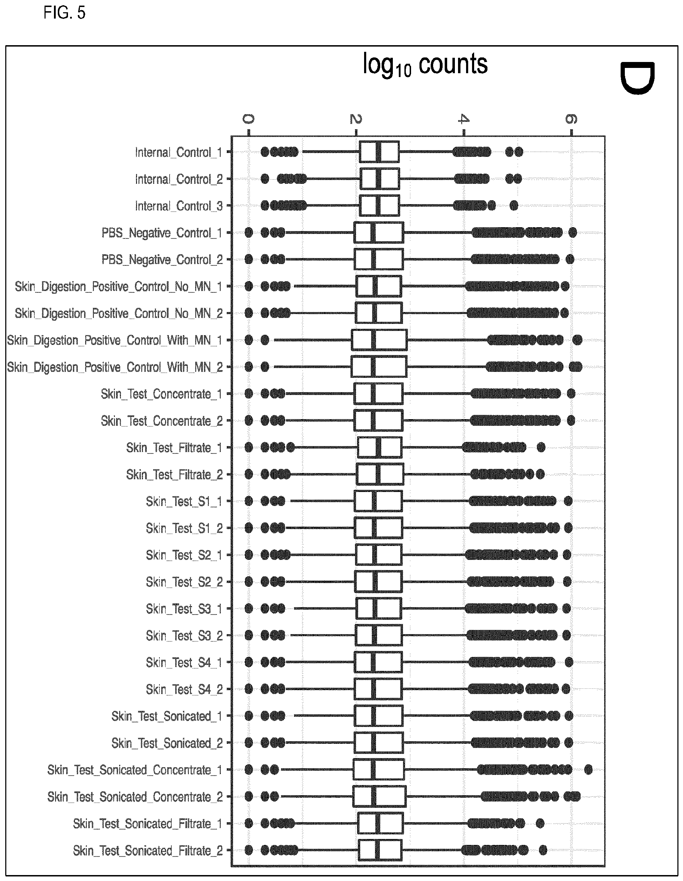

FIGS. 2-5 show the data quality-check and impact of the normalization. FIG. 2 shows the distribution of samples on the two first dimensions of a principal component analysis explaining 62.9% of the total variability. In this type of graphic, samples sharing the same miRNA expression pattern are expected to be close to each other. Despite a global satisfying structure (*i.e*., internal controls are close from each other; negative controls are located in the same area), technical replicates might not be in close proximity ("_1" and "_2" for a given condition). This is mainly due to the fact that there is a spread in signal distribution and in total number of read generated between samples (FIG. 3). Applying a housekeeping-guided quantile normalization allowed us to align samples both in term of total number of reads and in term of distribution (FIG. 5), which leads to an even more satisfying global sample picture, bringing together technical replicates (FIG. 4).

### Conclusion

Based on the FIGS. 2-5, it can be concluded that the quantile normalization followed by a housekeeping-guided adjustment is well-suited to pre-process HTG EdgeSeq miRNA whole transcriptome. The normalized data are used for further analyses.

### EXAMPLE 3 - Reproducibility

To assess the reproducibility of the results from Example 2 above, three aspects were evaluated:
(1) The technical reproducibility of the HTG sequencing platform focusing on the three internal control tracks corresponding to the same sample sequenced three times;
(2) The technical reproducibility of the assay focusing on the four *in vitro* skin samples for which two technical replicates we generated; and
(3) The biological reproducibility of the assay focusing on the four *in vitro* skin samples after combining the information for matched technical replicates.

### (1) Internal controls

To evaluate the reproducibility between internal controls, the pair-wise Pearson's correlation coefficient between each pairing of three controls provided by HTG was computed.

**Table 3:**

| Internal Control 1 | Internal Control 2 | Internal Control 3 | |
|---|---|---|---|
| --- | 0.97 | 0.969 | Internal Control 1 |
| --- | --- | 0.963 | Internal Control 2 |
| --- | --- | --- | Internal Control 3 |

Table 3 shows the pair-wise Person's correlation coefficients R2 when correlating one sample versus another.

As can be seen from the above table, the technical reproducibility of the HTG platform is good, achieving an average .97 correlation between. Note that the correlation is lower for lowly expressed miRNA.

### (2) Technical Reproducibility

Using the same approach as above, to evaluate the reproducibility between the technical replicates, the pair-wise Pearson's correlation coefficient was computed between each pair of technical replicates from skin test samples.

**Table 4:**

| Skin Test S1_1 | Skin Test S1_2 | Skin Test S2_1 | Skin Test S2_2 | Skin Test S3_1 | Skin Test S3_2 | Skin Test S4_1 | Skin Test S4_2 | |
|---|---|---|---|---|---|---|---|---|
| --- | 0.941 | 0.913 | 0.898 | 0.902 | 0.898 | 0.916 | 0.932 | Skin Test S1_1 |
| --- | --- | 0.914 | 0.901 | 0.903 | 0.901 | 0.914 | 0.931 | Skin Test S1_2 |
| --- | --- | --- | 0.915 | 0.927 | 0.922 | 0.897 | 0.913 | Skin Test S2_1 |
| --- | --- | --- | --- | 0.909 | 0.908 | 0.89 | 0.898 | Skin Test S2_2 |
| --- | --- | --- | --- | --- | 0.948 | 0.881 | 0.903 | Skin Test S3_1 |
| --- | --- | --- | --- | --- | --- | 0.875 | 0.905 | Skin Test S3_2 |
| --- | --- | --- | --- | --- | --- | --- | 0.916 | Skin Test S4_1 |
| --- | --- | --- | --- | --- | --- | --- | --- | Skin Test S4_2 |

Table 4 shows the pair-wise Person's correlation coefficients R2 when correlating one sample versus another.

As can be seen from the table, between .92 and .95 correlation between technical replicates from four samples was achieved (Skin_Test_S1 1 vs Skin_Test_S1 2; Skin_Test_S2 1 vs Skin_Test_S2 2; Skin_Test_S3 1 vs Skin_Test_S3 2; Skin_Test_S4 1 vs Skin_Test_S4 2).

### (3) Biological Replicates

After assessing for the reproducibility between technical replicates, they were combined for every single sample. To evaluate the reproducibility between biological replicates, the pair-wise Pearson's correlation coefficient between each pair of skin test samples was computed.

**Table 5:**

| Skin_Test_S1 | Skin_Test_S2 | Skin_Test_S3 | Skin_Test_S4 | |
|---|---|---|---|---|
| --- | 0.945 | 0.931 | 0.962 | Skin_Test_S1 |
| --- | --- | 0.952 | 0.943 | Skin_Test_S2 |
| --- | --- | --- | 0.926 | Skin_Test_S3 |
| --- | --- | --- | --- | Skin_Test_S4 |

Table 5 shows the pair-wise Person's correlation coefficients R2 when correlating one sample versus another. As can be seen from the table, a range of .93 and .96 correlation between biological replicates is obtained.

### Conclusion

Overall, both technical and biological reproducibility is good, with correlation greater than .93.

### EXAMPLE 4 - Reliability

To evaluate reliability, the data obtained for the digested skin without patch (i.e. positive control) was compared to digested skin with patch. The miRNA expression values in the two samples were compared and the Pearson's correlation computed.

The pair-wise Pierson's correlation coefficients R2 correlating the two samples was calculated to be 0.828., suggesting that the application of the patch allows for a reliable measurement of skin miRNAome.

### EXAMPLE 5 - Relevance

To evaluate relevance, the results we obtained in our four biological replicates were compared with biopsies obtained in the scope of the miRNA Atlas project (https://ccb-web.cs.uni-saarland.de/tissueatlas/). In a first step, 41 skin-enriched miRNA were identified using the 30 tissues screened in the miRNA Atlas. The range of accuracy was then evaluated in measuring these miRNAs with the microneedle patch.

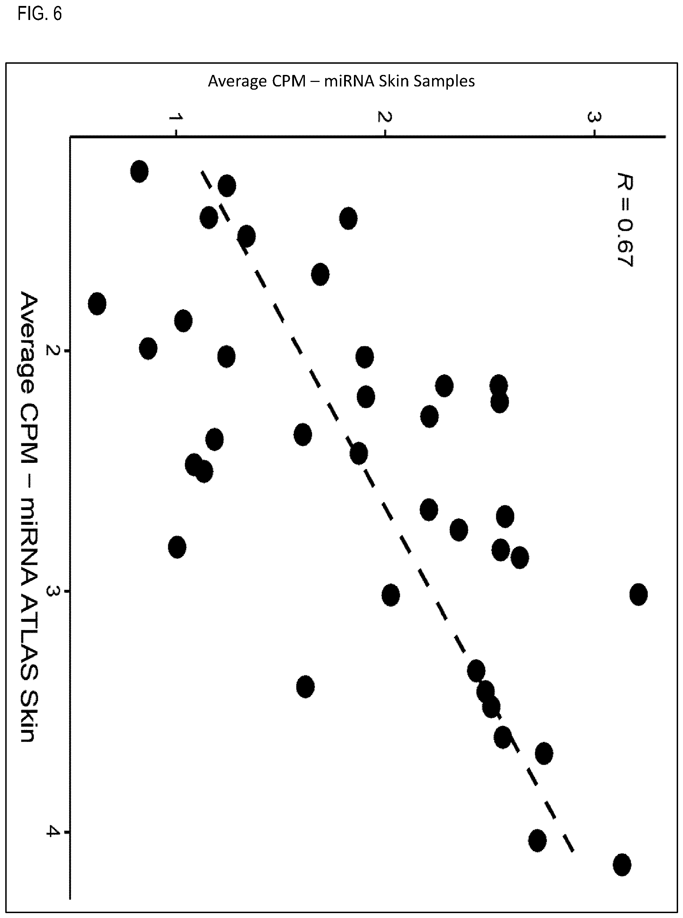

FIG. 6 shows a dot plot depicting expression level for skin-enriched miRNA in skin biopsies from the miRNA ATLAS and from the skin samples. The correlation is measured by the Pearson's correlation method. As seen from FIG. 6, there is an overall high correlation between the ATLAS and the sample data when considering skin-enriched miRNA set. This suggests that despite the inherent biological variability among the samples analyzed, the inventive process is both accurate and relevant.

### EXAMPLE 6 - Variations in Protocol

An effective protocol should limit the hands-on time during sample preparation, while still providing results as close as possible to the positive control (*i.e*., digested skin without microneedle). To evaluate the effect in variations of the protocol, pair-wise correlation analyses and clustering were performed. The Skin_Test samples (Ex. 4-7) overall clustered together. Moreover, they were shown to tend to correlate well with the positive controls. Since skin test samples were prepared with a PBS wash only, this demonstrates that a relatively simple protocol provides the right balance between accuracy and hands-on time.

In summary, no significant differences were observed due to the processing protocols. Even the simpler protocol of PBS wash only, with no concentration/filtration and no sonication provided satisfactory results.

### EXAMPLE 7 - Confirmation that Modulation in the miRNA Can be Detected

To investigate whether miRNA modulation could be detected through the patches, skin explants were treated with retinol and the miRNA profile was compared to skin explants treated with a placebo vehicle.

### Materials & Methods:

Abdominal skin biopsies were obtained from normal human adults undergoing plastic surgery. For the *ex vivo* experiment, four 1.13-cm² explants were punched in the skin sample under sterile conditions and placed on a thick filter paper soaked in culture medium (KGM^{™} Gold^{™} with supplements from Lonza) in a petri dish.

10µl of Retinol diluted at 0.1% in a mix of propylene glycol 30% and ethanol 70% was topically applied on the surface of two explants. In parallel, two control explants were treated with 10µl of the placebo vehicle: propylene glycol 30% and ethanol 70%. The skin explants were incubated for 24 hours and incubated under a 5% CO2 humidified atmosphere overnight at 37°C.

Microneedle patches were inserted into skin explant. The flat end of a plunger from a 5 ml syringe was pressed against the base of the microneedles array in order to insert them into the skin explant using firm pressure over 30 seconds. A clear tape was placed on each microneedle to fix them into skin explant. The microneedle patches were maintained in place for 120 minutes. To mimic *in vivo* conditions, the skin explants were incubated at 37 °C over the course of the experiment. After incubation, the microneedle patches of each sample were regrouped in the same tube and were washed with 500µL of PBS IX (Lonza) by inverting tube 4 times. The washing liquids were picked up for analysis.

The samples were sent to HTG Molecular to run an EdgeSeq miRNA whole transcriptome assay. After pre-processing and normalizing the data as previously described, a differential analysis was performed to detect miRNA with an altered expression due to the topical retinol treatment.

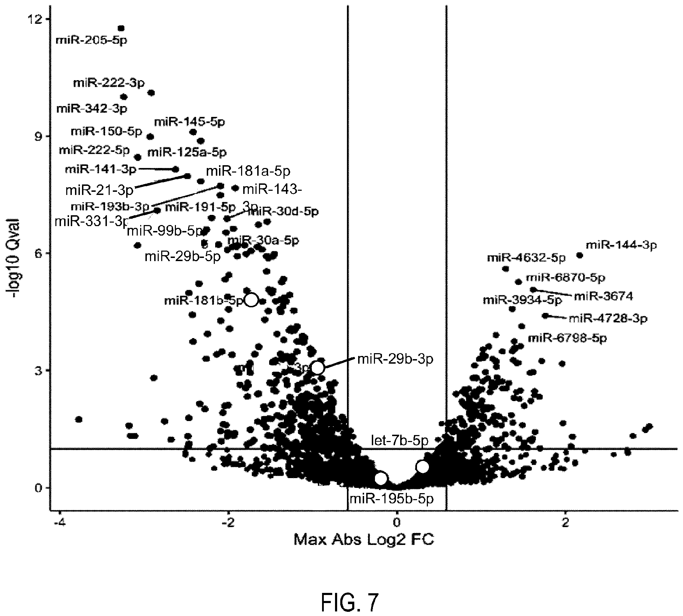

FIG. 7 shows a volcano plot depicting the results of the differential analysis evaluating the impact of retinol topic treatment. Each dot corresponds to a miRNA. The x-axis shows the log₂ fold-change in expression between control and treated samples. The y-axis shows the -log₁₀(p-value) for the Student *t*-test. One horizontal and two vertical lines are drawn on the graph to show significancy. Thus, dots which appear above the horizontal line and to the right of both vertical lines depict miRNA significantly up-regulated upon retinol treatment. Dots which appear above the horizontal line and to the left of both vertical lines depict miRNA significantly down-regulated upon retinol treatment. As can be seen from FIG. 7, 393 miRNA were down-regulated and 183 were up-regulated upon retinol topic treatment.

Additionally shown in FIG. 7, are miRNA previously shown to be down-regulated by retinol treatment (Parsa et al., Poster at AAD 2020), shown as hollow dots. Of interest, of five miRNAs previously shown to be downregulated by retinol treatment, four are part of the HTG panel. Of these four, two are detected as significantly downregulated by the patch (miR-181b-5p, miR-29b-3p) and one is slightly decreased (miR 195-5p).

This data demonstrates that the microneedle patch is capable of detecting modulations in the miRNA profile with miRNAs upregulated or downregulated after treatment with retinol.

## Claims

1. A method of quantifying miRNA in skin, the method comprising:
a. applying a swellable microprotrusion array to a region of skin to absorb interstitial fluid;
b. removing the microprotrusion array;
c. recovering miRNA from the interstitial fluid absorbed into the microprotrusion array; and
d. quantifying the miRNA.

2. The method of claim 1, wherein the microprotrusion array is applied to the skin for at least about 20 to about 180 minutes.

3. The method of any of claim 1-2, wherein recovering the miRNA form the microprotrusion array comprises washing the microprotrusion array.

4. The method of any of claim 1-3, wherein the microprotrusion array is washed with a buffer selected from the group consisting of phosphate-buffered saline, distilled water, and an RNA stabilization reagent to provide a washing liquid.

5. The method of any of claims 1-4, further comprising concentrating the washing liquid.

6. The method of any of claims 1-5, wherein the microprotrusion array comprises a plurality of microprotrusions composed of a swellable polymer composition.

7. The method of any of claims 1-6, wherein the swellable polymer composition is in its dry state hard and brittle sufficient to penetrate the stratum corneum of mammalian skin.

8. The method of any of claims 1-7, further comprising (e) comparing the quantified miRNA to a reference.

9. The method of any of claims 1-8, wherein quantifying the miRNA comprises next generation sequencing, transcriptome analysis, or quantitative polymerase chain reaction.

10. A method of monitoring epigenetic changes in skin as a result of exposure to a stimulus, the method comprising:
a. exposing a region of the skin of a subject to the stimulus;
b. applying a swellable microprotrusion array to at least a part of the region of skin to absorb interstitial fluid;
c. removing the microprotrusion array;
d. recovering miRNA from the microprotrusion array; and
e. analyzing the miRNA.

11. The method of claim 10, wherein the swellable microprotrusion array that is applied to at least a part of the region of skin to absorb interstitial fluid is the second swellable microprotrusion array, and the method further comprises
a. applying a first swellable microprotrusion array to a region of skin to absorb interstitial fluid prior to exposure to the stimulus, wherein the region of skin is the same region of skin to which the stimulus is exposed or a different region of skin in the same subject;
b. removing the microprotrusion array;
c. recovering miRNA from the microprotrusion array; and
d. analyzing the miRNA.

12. A method of monitoring epigenetic changes in skin as a result of exposure to a stimulus, the method comprising:
a. applying a first swellable microprotrusion array to a region of the skin of a subject to absorb interstitial fluid;
b. removing the first microprotrusion array;
c. recovering miRNA from the first microprotrusion array;
d. analyzing the miRNA from the first microprotrusion array;
e. exposing a region of skin which is either the same region of skin or a different region in the same subject to a stimulus;
f. applying a second swellable microprotrusion array to the region of skin exposed to the stimulus to absorb interstitial fluid;
g. removing the second microprotrusion array;
h. recovering miRNA from the second microprotrusion array; and
i. analyzing the miRNA from the second microprotrusion array.

13. The method of any of claims 10-12, wherein the stimulus comprises a composition.

14. The method of any of claims 10-13, wherein the composition comprises one or more dermatological active ingredients.

15. The method of any of claims 10-14, wherein exposure to the stimulus comprises topically applying the composition to the region of skin.

16. The method of any of claims 10-15, wherein the composition is in the form of a solution, suspension, lotion, cream, serum, gel, stick, spray, ointment, liquid wash, soap bar, shampoo, hair conditioner, paste, foam, powder, mousse, shaving cream, hydrogel, or film-forming product.

17. The method of any of claims 11-16, wherein the miRNA recovered from the microprotrusion array applied before administration of the composition is compared to the miRNA recovered from the microprotrusion array applied after administration of the composition.

18. The method of any of claims 11-17, wherein the composition is administered to a region of skin which is the same type of skin to which the first swellable microprotrusion array is applied.

19. The method of any of claims 11-18, wherein the stimulus is selected from the group consisting of UV light, pollution, irritants, allergens, temperature change, humidity change, and combinations thereof.

20. The method of any of claims 11-19, wherein the method further comprises a pretreatment step, wherein prior to application of the first swellable microprotrusion array to the region of the skin, the region of skin is exposed to a pretreatment stimulus.

21. The method of any of claims 12-20, further comprising
j. exposing a region of skin which is either the same region of skin or a different region in the same subject to a second stimulus;
k. applying a third swellable microprotrusion array to the region of skin exposed to the second stimulus to absorb interstitial fluid;
l. removing the third microprotrusion array;
m. recovering miRNA from the third microprotrusion array; and
n. analyzing the miRNA from the third microprotrusion array.
